# EUROPEAN PATENT SPECIFICATION

(11) **EP 0 715 849 B1**
(45) Date of publication and mention of the grant of the patent: **25.07.2001**
(21) Application number: 95118986.9
(22) Date of filing: 03.01.1992
(51) Int. Cl.: A61K 31/155, A61K 31/195

(54) **A method of inhibiting endotoxin induced effects**
Verfahren zur Reduzierung von Endotoxin induzierten Effekten
Méthode de réduction les effets induits par l'endotoxine

(30) Priority: 09.01.1991 SE 9100059
(43) Date of publication of application: 12.06.1996
(62) Divisional of application: 92850001.6
(73) Proprietor: Pharmacia Aktiebolag, 112 87 Stockholm (SE)
(72) Inventor: Grimfors, Christer, S-182 75 Täby (SE); Lampén, Ellinor, S-163 65 Spanga (SE); Lindgren, Svante, S-195 00 Märsta (SE); Sandberg, Göran, S-762 00 Rimbo (SE); Wahlén, Raymond, S-194 53 Upplands Väsby (SE); Westberg, Björn, S-185 41 Waxholm (SE)
(74) Representative: Tannerfeldt, Sigrid Agneta

(56) References cited:
- EP-A- 0 342 139
- GB-A- 1 253 830
- GB-A- 2 240 041
- US-A- 4 282 217
- US-A- 4 308 280
- US-A- 4 405 643
- STN International, File Medline, STN accession no. 88290981, J.V. Reynolds et al: "Immunomodulatory mechanisms of arginine", Surgery, vol 104, no. 2, August 1988, pages 142-151
- STN International, File BIOSIS, STN accession no. 91:55734, R.G. Kilbourn et al: "Reverseal of endotoxin-mediated shock by N-G methyl-L-arginine an inhibitor of nitric oxide synthesis", Biochem. Biophys. Res. Commun, vol 172, no. 3, 1990, pages 1132-1138

## Description

When manufacturing pharmaceuticals for parenteral use, one of the most important prerequisites is that the products included in the pharmaceutical are non-pyrogenic, i.e. that the endotoxin concentration of the pharmaceutical concerned is so low that only very small biological effects or no biological effects can be detected with conventional test systems (limulus tests = LAL or temperature increase in rabbits). Endotoxins are high molecular complexes associated with the outer cell wall of Gram-negative bacteria (e.g. E. Coli, Proteus or Salmonella), from which lipopolysaccharides (LPS) can be released (endotoxins, O-antigens) (Rietschel, E.T., et al., in Bacterial Endotoxins: Structure, Biomedical Significance and Detection with Limulus Amebocyte Lysate Test, pages 31-50, Alan R. Liss Inc., 1985).

Endotoxins are present in and are often the cause of the clinical symptoms in sepsis and in ARDS and DIC (adult respiratory distress syndrome and direct intravascular coagulation, respectively) (Zaren, B. and Hedstrand, U., Intensivvård, pages 63-64, Uppsala University, Reprocentralen HSC, 1989).

Subsequent to having treated patients suffering from e.g., septicemia with antibiotics, it is well known that the temperature of the patient will rise or that a further fever peak will occur, so-called Herxheimer's reaction, wherewith dead bacteria and parts thereof, including endotoxins, enter the blood circulation.

Clinical signs of the effect of endotoxins (the limit at which these can be shown is about 5 EU per kilo of body weight in rabbits and human beings) can sometimes be observed when pharmaceuticals and nutrient solutions are administered parenterally. In the case of human beings and rabbits, for instance, the clinical signs are manifested by a feverish state, due to the ability of the endotoxins to release endogenic pyrogens which influence the thermoregulatory center in the central nervous system. Other manifestations can also be observed in the central nervous system (Nowotny, B., Naturwissenschaft 58, pages 397-409, 1971). Such cardiovascular changes as hypotension and permeability changes in arteriole and venules, for instance, may explain certain important organ changes which often occur in Gram-negative sepsis (Zaren, B. and Hedstrand, U., Intensivvård, pages 63-64, Uppsala University, Reprocentralen HSC, 1989; Nowotny, B., Naturwissenschaft 58, pages 397-409, 1971; Gilbert, R.P., Physiol. Rev. 40, 245, 1960; Vick, J.A., Am. J. Phys. 200, 944, 1964).

Those depyrogenizing methods which can be applied in vitro today are based on two principal techniques, namely a) to guard against endotoxin contamination and b) to remove endotoxins during formulation.

It is difficult to carry out the first method a) strictly, because it is necessary for aseptic conditions to prevail during the whole of the formulating process and also during the preparation of starting materials. The second method b) has resulted in the development of different filtering methods; these methods include the use of asbestos filters, ion exchangers, and have involved adsorption on activated carbon or on barium sulfate suspensions, gamma radiation, filtration through membranes having an exclusion limit ranging from 100,000 Daltons to 0.1 micron of endotoxin aggregate, the supply of amebocytlysate and the removal of the gel formed, and also the use of ultrafilters having an exclusion limit of 10,000 Daltons for filtering-out non-aggregated endotoxins. At the present time, ultrafiltration is primarily applied industrially, whereas the other methods have been abandoned, with the exception of asbestos filtration. Two depyrogenizing methods, namely autoclaving alone or in combination with extremely low pH-values, now have limited value because of their low efficiency and because of damage caused to the products (Mosier, L.D., et al. J. Parent. Sci. and Technol., Vol. 41, No. 1, pages 21-25, 1987). The ultrafiltration method, however, results in high production costs, because of the expensive material and high working costs involved. Furthermore, the equipment used is often highly space-consuming and often of doubtful efficiency, resulting in floating exclusion limits and enabling endotoxins to pass through the filters to some extent.

One particular problem in this regard is the assaying of endotoxins in biologically active substances, such as coagulation factor 2 (prothrombin), for instance, or when the sample material is highly restricted but has a very high biological potency, there excluding the use of both the limulus test and experimental animals.

So-called plasmapheresis and hemoperfusion through filters that contain an immobilized product of polymyxin B have been tested in vivo for the purpose of removing endotoxins from the blood path.

Methyl arginine is used as a competitive inhibitor of the ribosylation of ADP, which is necessary in order for endotoxins and cholera toxins to take effect and instigate diarrhea (Moss. J., Garrison, S., Oppenheimer, N.J., Richardsson, S.H., J. Biol. Chem., Vol. 254, No. 14, pages 6270-6272, 1979).

In the case of liver diseases caused by trauma, shock or surgery, it is stated in European Patent Specification No. EP-A-0059775 that a nutrient solution which contains, inter alia, L-arginine, malic acid, malate, L-asparaginic acid, glucose and carnitine has a protective effect on the liver as a result of stimulating the citrate and urea cycles and therewith lowering the ammonium ion concentrations and phenol concentrations in serum.

Patent Specification US-A-4282217 teaches a method of increasing the specificity and therewith the effect of corticosteroids, by combining these with esters of, for instance, methyl arginine or ethyl arginine and therewith obtaining a synergistic effect. Arginine esters were used in quantities of up to 6.3 mg per kilo of body weight in experiments on rats.

Patent Specification US-A-4308280 proposes the use of arginine esters as a medicament against endotoxin induced pulmonary edema. This clinical picture is highly similar to the ARDS condition earlier mentioned. The claims of this application recite methyl arginine dosages of from 0.25 mg up to 100 mg/kilogram of rat body weight.

### DESCRIPTION OF THE INVENTION

The invention relates to the use of a compound according to formula I 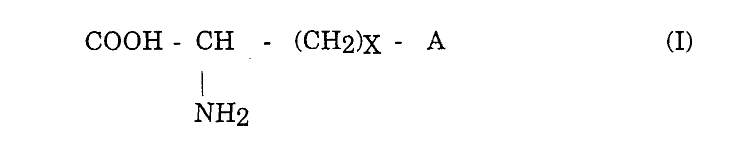 in which X is an integer of from 2 to 5 and
A signifies - NH - C(=NH) - NH₂ or - CH₂ - NH₂ for the preparation of a medicament for treating endotoxin-induced effects, particularly for suppressing endotoxin-induced pyrexia.

The medicament is preferably administered orally, intravenously, intramuscularly, intracutaneously or intraperitoneally in an amount of 10-800 mg/kg body weight and hour.

By "arginine or structurally related substances" is meant in the following compounds according to formula (I).

Other features of the invention will be apparent from the following description and from the claims.

The use of the inventive compounds will now be exemplified with the aid of a number of test examples, although it will be understood that these examples do not limit the scope of the invention.

### Example 1

5 ng/ml of endotoxins from E. Coli (corresponding to 25 EU/ml endotoxins) together with various amino acids were injected into three live rabbits. The pyrogen reaction was assayed by recording the rectal temperature of the rabbits. The sum of the temperature increases is recited in Table 1. (One to four such experiments were carried out with each amino acid.) The result shows clearly that arginine does not result in an increase in temperature of the animals, as distinct from the other amino acids used in the test series (Table 1).

### Example 2

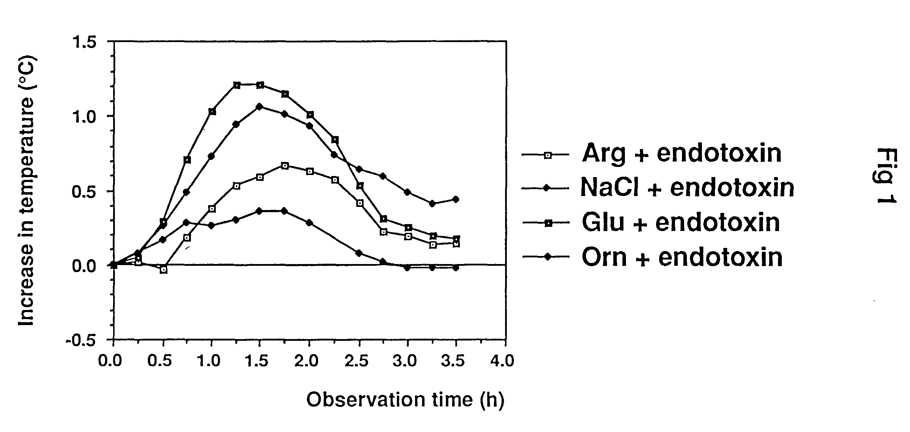

When administering a constant infusion of arginine solution (1600 mg/kg and hour) together with ornithine chloride solution (1000 mg/kg and hour) to 8 and 5 rabbits, respectively, over a period of about six hours, preceded by a bolus dosage of endotoxin (500 EU per kilo body weight), it was noted that the temperature development of these animals was significantly lower than the temperature development of two reference groups (6 and 5 rabbits, respectively), which in addition to a bolus dosage of endotoxins corresponding to 500 EU per kilo body weight were also constantly infused with physiological sodium chloride solution (0.9%), and 5% glucose solution, respectively, over a period of about six hours (see Figure 1 and Table 2).

### Table 2

The change in temperature of the rabbits 3.5 hours after injecting endotoxins in an amount corresponding to 500 EU/kg body weight and subsequent constant infusion corresponding to 20 ml/kg body weight of arginine, ornithine chloride, 0.9% sodium chloride solution and 5% glucose solution. The mean value recited in the Table relates to the area between the initial temperature and the fever chart.

| Group | Mean Value ± SEM | n |
|---|---|---|
| a. Arginine | 1.15 ± 0.35 | 8 |
| b. Ornithine chloride | 0.58 ± 0.24 | 5 |
| c. 0.9% NaCl | 2.15 ± 0.24 | 6 |
| d. 5% glucose | 2.22 ± 0.17 | 5 |
| a/c p<0.05 | | |
| a/d p<0.05 | | |
| a/c p<0.05 | | |
| b/d p<0.05 | | |

a/c p<0.05
a/d p<0.05
a/c p<0.05
b/d p<0.05

It is evident from the experiments disclosed in the Test Examples that:
- Among the amino acids tested in Table 1, arginine eliminates the temperature increasing effect of the endotoxins in vivo.
- In the case of constant infusion, arginine and ornithine in vivo are able to eliminate the temperature increasing effect of the endotoxins (Table 2).

The temperature inhibition corresponds to a general endotoxin inhibition. Thus, freely dissolvable arginine and ornithine, together with structurally-related substances, can be used to eliminate endotoxins in conditions of Gram-negative sepsis with endotoxin shock and ARDS and DIC development. Dosages of 50 mg per kilogram body weight and hour have been found to produce an effect on rabbits. Much higher dosages are required for human use, e.g. dosages of between 5-280 grams per day, suitably under continuous infusion (10-800 mg/kg per hour). (LD₅₀ for rats of Arg. HCl is 3.1 g/kg body weight as a single dosage. Milne, M.D., Pharmacology of Amino Acids. Clinical Pharmacology and Therapeutics, Vol. 9, pages 484-516, 1968). The shock condition is caused by the endotoxins that are produced by the bacteria and not by the bacteria themselves. Endotoxins are present in the blood path even after elimination of the bacteria by the body's own antibacterial system or by means of exogenically administered antibacterial substances. A combined treatment with arginine or structurally-related substances, intravenously/orally in high dosages, and an antibacterial treatment with an appropriate antibiotic is thus clearly indicated.

**Table 1**

| Pyrogen reaction in rabbits when testing earlier pyrogen-free amino acids in solutions to which 10 EU endotoxins were added for each 10 ml of solution. | | | | | | |
|---|---|---|---|---|---|---|
| Amino Acid | Conc. (g/l) | Dos. (ml/kg body weight) | Total Temp. Inc. (°C) of three rabbits | | | |
| Arginine | 25 | 10 | 0.70 | 0.65 | 0.55 | 0.70 |
| Alanine | 20 | 10 | 3.40 | 2.50 | 2.75 | |
| Asparaginic acid | 5 | 10 | 2.85 | | | |
| Phenyl alanine | 25 | 10 | 2.25 | | | |
| Glutamic acid | 10 | 10 | 2.60 | | | |
| Glycine | 20 | 10 | 2.80 | 2.65 | 2.55 | 2.70 |
| Histidine | 15 | 10 | 3.60 | | | |
| Isoleucine | 20 | 10 | 3.40 | 4.00 | 2.85 | 2.00 |
| Leucine | 20 | 10 | 2.60 | 2.60 | 2.65 | 2.60 |
| Lysine chloride | 20 | 10 | 1.95 | 1.60 | 3.05 | 2.10 |
| Methionine | 10 | 10 | 2.05 | 3.40 | 1.80 | 2.95 |
| Proline | 10 | 10 | 3.15 | 4.40 | | |
| Serine | 10 | 10 | 3.75 | 3.05 | 0.45 | 3.30 |
| Tryptophan | 10 | 10 | 2.70 | 3.95 | 3.50 | 2.60 |
| Tyrosine | 0.5 | 10 | 3.25 | 3.85 | 2.40 | 3.20 |
| Threonine | 15 | 10 | 2.75 | 4.15 | 2.35 | 2.80 |
| Valine | 20 | 10 | 3.95 | 3.40 | 1.70 | 1.95 |

## Claims

1. Use of a compound according to formula I 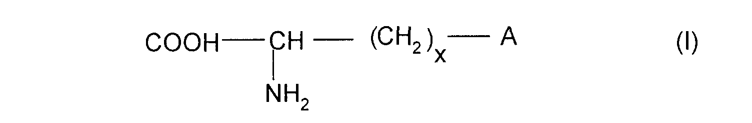 in which x is an integer of from 2 to 5 and
A signifies - NH - C(=NH) - NH₂ or - CH₂ - NH₂ in the preparation of a medicament for the treatment of endotoxin induced effects which is to be administered by infusion in a total amount of 5 to 280 g/day.

2. The use of a compound according to formula (I) in the preparation of a medicament for reducing endotoxin induced fever.

3. The use according to claim 2 characterized in that the medicament is administered orally.

## Patentansprüche

1. Verwendung einer Verbindung gemäß Formel I 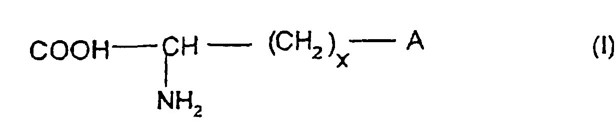 in der x eine ganze Zahl von 2 bis 5 ist und
A für -NH-C(=NH)-NH₂ oder -CH₂-NH₂ steht, zur Herstellung eines Arzneimittels, das durch Infusion in einer Gesamtmenge von 5 bis 280 g/Tag verabreicht werden soll, für die Behandlung von durch Endotoxin induzierten Effekten.

2. Verwendung einer Verbindung gemäß Formel I zur Herstellung eines Arzneimittels zur Senkung von durch Endotoxin induziertem Fieber.

3. Verwendung nach Anspruch 2, dadurch gekennzeichnet, dass das Arzneimittel oral verabreicht wird.

## Revendications

1. Utilisation d'un composé selon la formule 1 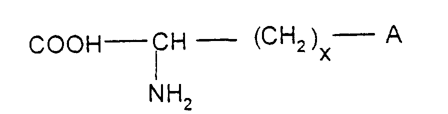 dans laquelle x est un nombre entier de 2 à 5 et
A signifie - NH - C(=NH) - NH₂ ou- CH₂ - NH₂
dans la préparation d'un médicament pour le traitement d'effets induits par l'endotoxine qui doit être administré par perfusion dans une quantité totale de 5 à 280 g/jour.

2. Utilisation d'un composé selon la formule (1) dans la préparation d'un médicament pour réduire la fièvre induite par l'endotoxine.

3. Utilisation selon la revendication 2, caractérisée en ce que le médicament est administré par voie orale.
